# EUROPEAN PATENT APPLICATION

(11) **EP 4 813 968 A1**
(43) Date of publication of application: **30.09.2026**
(21) Application number: 25166298.7
(22) Date of filing: 26.03.2025
(51) Int. Cl.: C07C 45/48, C07C 49/04, C07C 1/22, C07C 9/14

(54) **KETONE PRODUCTION**

(71) Applicant: Evonik Operations GmbH, 45128 Essen (DE); Ruhr-Universität Bochum, 44801 Bochum (DE)
(72) Inventor: SIVENDRAN, Nardana, 45721 Haltern am See (DE); MERZ, Juliane, 44139 Dortmund (DE); QUANDT, Thomas, 45772 Marl (DE); GOOSEN, Lukas, 45529 Hattingen (DE); SAVIOZZI, Mattia, 44137 Dortmund (DE); JANNING, Henric, 44623 Herne (DE)
(74) Representative: Evonik Patent Association

(57) **Abstract**

The present invention relates to a method of producing a ketone **K1** of the Formula **I**:

R1-C(=O)-R2 Formula **I,**

wherein a carboxylic acid R1-COOH and a carboxylic acid R2-COOH are reacted in the presence of a catalyst composition **X** to give **K1** and CO₂; and
wherein R1 and R2 is independently a hydrocarbon group, preferably R1 and R2 is an organic radical selected from the group consisting of unsubstituted and mono- or polysubstituted, branched and straight-chain alkyl radicals, cycloalkyl radicals, alkenyl radicals having one or more double bonds, alkynyl radicals having one or more triple bonds, aryl radicals, alkylaryl radicals, arylalkyl radicals, arylalkenyl radicals, alkyloxyalkyl radicals, hydroxyalkyl radicals, aminoalkylradicals and alkylthioalkyl radicals; and
wherein the catalyst composition **X** comprises:
- at least one precious metal; and/or
- at least one mixed oxide.

## Description

### FIELD OF THE INVENTION

The present invention relates to a method of producing ketones from carboxylic acids. In particular, the method includes the use of a specific catalyst composition comprising at least one precious metal; and/or at least one mixed oxide comprising zirconium oxide.

### BACKGROUND OF THE INVENTION

There are various well-known processes for the production of ketones from a variety of different raw materials. These processes for example include oxidation of secondary alcohols, Friedel-Crafts acylation, reaction of acid chlorides with organic cadmium compounds, acetoacetic ester synthesis and decarboxylation from acids, and also ketonisation of organic acids among others.

It is however, also well-known that there are several problems associated with these known processes to produce ketones. These include problems such as the unavailability and/or high cost of raw materials, the requirement of multi-stage processing, the low conversion of raw materials and/or the low selectivity of the desired ketones, the production of corrosive or hard-to-separate products amongst other problems of the known processes of ketone production.

In fact, the most used ketone manufacturing processes include the reaction of various different reactants at a specified temperature and pressure ranges in the presence of different catalysts. US4528400, US4874899, US4570021, US4060555, US3966822, US3466334, and US3453331 are some examples of prior art that disclose a process of synthesizing ketones from a variety of starting products at a variety of temperature and pressure conditions.

A great deal of attention has been given to the selection of feed materials and the process parameters such as temperature and pressure in connection with the production of ketones. However, little, if any, attention has been given to evaluating the role of the catalyst in the ketone preparation process or to selecting or preparing the catalyst for the purpose of maximizing the conversion and selectivity of the feed material to the desired ketone product. US6369276 for example discloses a catalyst and catalyst structure for use in the production of organic compounds such as ketones, which has been formulated to optimize the conversion of feed reactants and selectivity to the desired ketone. However, the catalyst production is complicated.

Accordingly, there is a need in the art for a catalyst useful in the production of ketones from simple starting materials which not only allows the reaction to proceed, but which also optimizes the conversion and selectivity of the reaction to the desired ketone.

### DESCRIPTION OF THE INVENTION

The method according to any aspect of the present invention solves the problems above by using at least one specific ketonisation catalyst for the ketonisation of carboxylic acids to produce a corresponding ketone. In particular, the ketonisation catalyst is a special catalyst composition that comprises at least one precious metal; and/or at least one mixed oxide and the mixed oxide comprises zirconium dioxide and silicon dioxide and the mass ratio of zirconium dioxide to silicon dioxide in the mixed oxide is 86:14 to 99.9:0.1. It was found that, surprisingly, this specific ketonisation catalyst according to any aspect of the present invention catalyses ketone production from carboxylic acid. In particular, a high yield of ketone was obtained from a simple and cost efficient raw material - carboxylic acid. The ketone can be obtained in high yield and purity. Furthermore, it is possible to use smaller catalyst amounts compared to the related art and/or realize shorter reaction times. Moreover, the reactions are catalyzed at lower temperatures.

According to one aspect of the present invention, there is provided a method of producing a ketone **K1** of the Formula **I**:

R1-C(=O)-R2 Formula **I,**

wherein a carboxylic acid R1-COOH and a carboxylic acid R2-COOH are reacted in the presence of a catalyst composition **X** to give **K1** and CO₂; and
wherein R1 and R2 is independently a hydrocarbon group, preferably R1 and R2 is an organic radical selected from the group consisting of unsubstituted and mono- or polysubstituted, branched and straight-chain alkyl radicals, cycloalkyl radicals, alkenyl radicals having one or more double bonds, alkynyl radicals having one or more triple bonds, aryl radicals, alkylaryl radicals, arylalkyl radicals, arylalkenyl radicals, alkyloxyalkyl radicals, hydroxyalkyl radicals, aminoalkylradicals and alkylthioalkyl radicals; and wherein the catalyst composition **X** comprises:
   - at least one transition metal; and/or
   - at least one mixed oxide.

In particular, the carboxylic acid according to any aspect of the present invention has a formula R1-COOH or R2-COOH wherein R1 and R2 is independently a hydrocarbon group. In particular, suitable carboxylic acids are monofunctional or else difunctional or higher-functional carboxylic acids. Monocarboxylic acids contemplated include saturated and preferably unsaturated carboxylic acids such as benzoic acid, cyclohexanecarboxylic acid, 2-ethylhexanoic acid, caproic acid, caprylic acid, capric acid, lauric acid, natural and synthetic fatty acids, in particular acrylic acid, methacrylic acid, dimeric acrylic acid or crotonic acid. Suitable dicarboxylic acids are phthalic acid, isophthalic acid, tetrahydrophthalic acid, hexahydrophthalic acid, cyclohexanedicarboxylic acid, maleic acid, fumaric acid, malonic acid, succinic acid, glutaric acid, adipic acid, azelaic acid, pimelic acid, suberic acid, sebacic acid, dodecanedioic acid, and hydrogenated dimeric fatty acids.

More in particular, R1 or R2 is an organic radical, a hydrogen, an optionally unsaturated, optionally substituted, optionally one or more heteroatoms containing straight chain or branched alkyl group having up to 55 carbon atoms; an optionally unsaturated, optionally substituted, optionally one or more heteroatoms containing C5-8 cycloalkyl group; an optionally substituted aryl or heteroaryl group; or an optionally substituted benzyl group. In one example, the alkyl group is optionally interrupted by an oxygen atom or by an internal ester group. In one example, the alkyl group has 1 to 50 carbon atoms, particularly 10 to 50 carbon atoms, more particularly 4 to 12 carbon atoms.

A particular substituent of the above defined groups is a hydroxyl group, especially an [alpha]-hydroxyl group.

In one example, the carboxylic acid is hexanoic acid, octanoic acid, stearic acid, oleic acid, linoleic acid, 27-stearoyloxy-heptacosanoic acid, 27-linoleoyloxy-heptacosanoic acid, [alpha]-hydroxy-stearic acid, lactic acid, retinoic acid, salicylic acid or ferulic acid. In particular, the carboxylic acid is an alkanoic acid comprising 1 to 22 carbon atoms, particularly a hexanoic acid.

The catalyst composition **X** comprises at least one transition metal and/or at least one mixed oxide, where the mixed oxide comprises zirconium dioxide and silicon dioxide. In one example, the catalyst composition according to any aspect of the present invention comprises only a mixed oxide comprising zirconium dioxide and silicon dioxide. In particular, the mass ratio of zirconium dioxide to silicon dioxide in the mixed oxide is 50:50, 51:49, 52:48, 53:47, 54:46, 55:45, 56:44, 57:43, 58:42, 59:41, 60:40, 61:39, 62:38, 63:37, 64:36, 65:35, 66:34, 67:33, 68:32, 69:31, 70:30, 71:29, 72:28, 73:27, 74:26, 75:25, 76:24, 77:23, 78:22, 79:21, 80:20, 81:19, 82:18, 83:17, 84:16, 85:15, 86:14, 87:13, 88:12, 89:11, 90:10, 91:9, 92:8, 93:7, 94:6, 95:5, 96:4, 97:3, 98:2, or 99:1. More in particular, the mass ratio of zirconium dioxide to silicon dioxide in the mixed oxide is 50:50 to 99:1, 55:45 to 99:1, 60:40 to 99:1, 70:30 to 99:1, 80:20 to 99:1, 90:10 to 99:1, 50:50 to 95:5, 55:45 to 95:5, 60:40 to 95:5, 70:30 to 95:5, 80:20 to 95:5, 90:10 to 95:5. Even more in particular, the mass ratio of zirconium dioxide to silicon dioxide in the mixed oxide is 86:14 to 99.9:0.1, particularly 90:10 to 97:3, more particularly 95:5. The stated mass ratio excludes, for example, silicon dioxide doped with zirconium dioxide. The mass ratio is calculated on the basis of the zirconium and silicon compounds used for the mixed oxide.

Catalyst compositions with mixed oxides outside of the specified mass ratio exhibit significantly lower activities. Moreover, the selectivity of the ketone formation decreases when the specified mass ratio is outside of that used according to any aspect of the present invention.

The term 'mixed oxide' as used herein is understood as meaning a composition which comprises at least zirconium dioxide and silicon dioxide. Zirconium dioxide and silicon dioxide are not present in the mixed oxide as concrete compounds, but serve merely as a base for calculating the mass ratios. The mixed oxide is obtained by calcination. The mixed oxide therefore does not constitute a physical mixture of zirconium dioxide and silicon dioxide, but a chemical mixture comprising at least silicon and zirconium cations with a unique crystal structure. In this regard, a physical mixture which comprises at least the two oxides and has not been calcined does not constitute a mixed oxide for the purposes of the invention. Nor is zirconium dioxide-doped or coated silicon dioxide included.

In particular, the mixed oxide comprises zirconium dioxide and silicon dioxide or consists of these two oxides. The fraction of the sum of zirconium dioxide and silicon dioxide in the mixed oxide is particularly at least 20% by weight and more particularly at least 30% by weight, even more particularly 50% by weight and very particularly 95% by weight, in each case based on the total weight of the mixed oxide. The mixed oxide particularly consists of zirconium dioxide and silicon dioxide.

The catalyst compositions according to any aspect of the present invention comprises the mixed oxide. In one example of catalyst compositions according to any aspect of the present invention, the catalyst composition comprises transition metals. In particular, the transition metals are either rare metals, precious metals or mixtures thereof. In this example, a support for the transition metal can be present which does not consist of the mixed oxide. Particularly, the support does not comprise the mixed oxide or consist of it. The mixed oxide as support, and also the inert support, can be present as powders or as mouldings, preference being given to these supports as mouldings. It is likewise preferred that the mixed oxide is present as moulding if the mixed oxide does not function as precious metal support.

Suitable mouldings are beads, extrudates, tablets, granules and pellets. The conversion of powders to mouldings is described for example in chapter 9 "Shaping of Solid Catalysts" in the book "Synthesis of Solid Catalysts", ed K. P. de Jong, Wiley-VCH Verlag GmbH & Co. KGaA, Weinheim, Germany (2009).

The weight-based ratio of mixed oxide to transition metal, particularly precious metal for all catalysts used according to any aspect of the present invention can be 99.9:0.1 to 50:50, particularly 99.5:0.5 to 80:20 and more particularly 99:1 to 90:10.

In one example, where the catalyst composition according to any aspect of the present invention comprises a precious metal and the precious metal is supported, the fraction of precious metal, based on the total weight of precious metal and support, can be 0.01 to 5% by weight, particularly 0.05 to 2.5% by weight and more particularly 0.3 to 2.0% by weight. The precious metal can be distributed on or within the support.

The precious metal of the catalyst composition according to any aspect of the present invention may be selected from the group consisting of ruthenium, rhodium, palladium, osmium, iridium, platinum and mixtures thereof, with ruthenium, palladium and platinum being preferred and ruthenium and palladium being particularly preferred and palladium being very particularly preferred. In the series of the three precious metals ruthenium, palladium and platinum, platinum is less suitable because of its comparatively low selectivity with regard to CDON. The precious metal can be present as powder (unsupported) or in supported form. The precious metals can be present as elemental metals or in the form of their oxides, preference being given to elemental metals.

A silicon dioxide as solid is understood as meaning a powder which does not form a stable dispersion in water. The silicon dioxide as solid particularly has a particle size d50 of less than 500 µm. In particular, the particle size is less than 500 µm, this upper limit being determined by means of sieve analysis (mesh of 500 µm; this upper limit, determined by means of sieve analysis, is consequently a maximum value and not an average value d50 ). Likewise, the solid particularly has a particle size d50 of greater than 100 nm. The particle size d50 is particularly greater than 500 nm and more particularly greater than 1 µm. The particle size d50 will be ascertained by laser diffraction in accordance with ISO 13320:2009. For the measurement operation, solid (according to manufacturer's instructions) is placed into the Scirocco 2000 dispersing unit of a Malvern Mastersizer 2000. The pressure selected is 1 bar and the measurement is carried out.

Colloidal solutions of SiO₂ such as Ludox (Grace Davison) and Köstrosol (CWK Bad Köstritz) are not preferred. These types of colloidal solutions generally comprise individual SiO₂ particles with a size d50 between 5 and 30 nm. In order to ascertain the particle size of SiO₂ in a colloidal solution, a Zetasizer of the nano line from Malvern is used. The measurement is carried out at room temperature.

In particular, the silicon dioxide is produced by means of pyrogenic methods. The process is known to the person skilled in the art, e.g. from the series of papers "Fine Particles" No. 11 (7th Edition, 2003), company journal of Degussa AG.

The zirconium compound in the catalyst composition X according to any aspect of the present invention is selected from zirconium dioxide, zirconium hydroxide, zirconium acetate, zirconium nitrate, zirconium oxychloride, ammonium zirconium carbonate or mixtures thereof. In particular, the zirconium compound in the catalyst composition **X** according to any aspect of the present invention is zirconium dioxide, zirconium hydroxide or mixtures thereof.

Zirconium hydroxide is understood as meaning zirconium (IV) hydroxide.

It is likewise preferred to select the zirconium compound from a mixture comprising A and B. In this connection, A comprises zirconium dioxide, zirconium hydroxide and mixtures thereof. B is selected from zirconium acetate, zirconium nitrate, zirconium oxychloride, ammonium zirconium carbonate and mixtures thereof. The fraction of zirconium from A is particularly at least 85 mol %, particularly at least 90 mol %, based on the sum of zirconium from A and B.

As a result of the calcination, the zirconium compounds are reacted at least partially to give zirconium dioxide. In this regard, zirconium compounds, apart from zirconium dioxide itself, are referred to as precursors.

Preferably, the person skilled in the art selects time conditions through which at least 50 mol %, particularly at least 90 mol %, more particularly 95 mol % and even more particularly 100 mol %, of the zirconium compounds, in each case based on the sum of all zirconium compounds, have reacted to give zirconium dioxide.

The person skilled in the art can adjust the size of the BET surface area of the mixed oxide by known measures in order to obtain, for example, surface areas of less than 155 m² /g. The higher the fraction of silicon dioxide in the mixed oxide, the higher the surface area will be. Consequently, at most 14% by weight of silicon dioxide, based on the total weight of the mixed oxide, are present. Furthermore, the calcination temperature influences the surface area: The lower the set temperature, the higher the surface area will be. Consequently, the calcination temperature is not below 300° C., preferably not below 400° C. Moreover, preferably no polymers for enlarging the surface area, as is described for example in EP-A-2108631 (US 2009/0255402), are present. By means of a few experiments, the person skilled in the art is able to undertake an adjustment of the surface area by virtue of the specified parameters.

A method of producing the catalyst composition **X** according to any aspect of the present invention, is described in EP3002058A1. Based on the methods disclosed in EP3002058A1, a skilled person would be able to produce the catalyst composition **X** that will be suitable for production of at least one ketone **K1** from carboxylic acids according to any aspect of the present invention.

Any ketone may be produced according to any aspect of the present invention depending on the starting carboxylic acids. In particular, the ketone **K1** has a structure R1-C(=O)-R2, where R1 and R2 can be a variety of carbon-containing substituents. Ketones contain a carbonyl group -C(=O)- (which contains a carbon-oxygen double bond C=O). In particular, the ketone may comprise at least three carbon atoms. More in particular, the ketone in the method according to any aspect of the present invention may be a C₃ to C₃₁ ketone. In one example, the ketone is at least one 6-undecanone which is a dialkyl ketone with formula (CH₃(CH₂)₄)₂CO and/or the carboxylic acid is a hexanoic acid.

In particular, the catalyst composition **K1** according to any aspect of the present invention is specifically suited for producing ketones from a carboxylic acids. The catalyst composition **K1** comprises at least a mixed oxide of zirconium dioxide and silicon dioxide. In particular, the catalyst composition according to any aspect of the present invention comprises only a mixed oxide of zirconium dioxide and silicon dioxide with a mass ratio of 50:50 to 99:1, particularly with a mass ratio of 86:14 to 99.9:0.1, more particularly 90:10 to 97:3, even more particularly 95:5.

It would be within the knowledge of a skilled person to determine the suitable conditions for the use of the different ketonization catalysts in the method according to any aspect of the present invention. In particular, the temperature has to be at least 300°C for the carboxylic acids to be converted to the corresponding ketone. More in particular, the temperature may be maintained at about 300, 325, 350, 355, 360, 365, 370, 375, 380, 385, 390, 395, or 400°C during the method according to any aspect of the present invention. Even more in particular, the reaction temperature may be 350-420, 350-415, 350-410, 350-405, 350-400, 350-395, 350-390, 350-385, 350-380, 350-375, 350-370, 350-365, 350-360, 355-420, 355-415, 355-410, 355-405, 355-400, 355-395, 355-390, 355-385, 355-380, 355-375, 355-370, 355-365, 355-360, 360-420, 360-415, 360-410, 360-405, 360-400, 360-395, 360-390, 360-385, 360-380, 360-375, 360-370, 360-365, 365-420, 365-415, 365-410, 365-405, 365-400, 365-395, 365-390, 365-385, 365-380, 365-375, 365-370, 370-420, 370-415, 370-410, 370-405, 370-400, 370-395, 370-390, 370-385, 370-380, 370-375, 375-420, 375-415, 375-410, 375-405, 375-400, 375-395, 375-390, 375-385, 375-380, 380-420, 380-415, 380-410, 380-405, 380-400, 380-395, 380-390, 380-385, 385-420, 385-415, 385-410, 385-405, 385-400, 385-395, 385-390, 390-420, 390-415, 390-410, 390-405, 390-400, 390-395, 395-420, 395-415, 395-410, 395-405, 395-400, 400-420, 400-415, 400-410, or 400-405 °C. More in particular, the method according to any aspect of the present invention is carried out at a temperature between 350°C and 390°C.

The method according to any aspect of the present invention may comprise a further step of extracting the ketone produced. Any method known in the art may be used to extract the ketone. In particular, distillation or basic washing may be used to extract the ketone.

The term "contacting", as used herein, means bringing about direct contact between the carboxylic acid with at least one catalyst composition according to any aspect of the present invention in the medium in step (a). For example, the carboxylic acid, and the catalyst composition may be in different compartments and brought together to produce the ketone **K1** according to any aspect of the present invention.

Under suitable conditions, the ketone **K1** is converted to the corresponding alkane **A1** in the presence of at least the catalyst composition **X**. A skilled person would understand what suitable conditions are required to produce the corresponding alkane **A1** from the **K1** produced according to any aspect of the present invention. In particular, contact with the catalyst composition **X** has to be maintained longer that the ketone **K1** converts to the corresponding alkene.

A skilled person would understand the suitable conditions for alkane production from a ketone. In particular, a suitable higher pressure and temperature would be necessary for the reaction to take place.

In particular, when the ketone **K1** comes in contact with the catalyst composition **X**, catalytic hydrogenation of the ketone **K1** takes place and the corresponding linear alkanol is produced, particularly a molecule of hydrogen is added across the carbon-oxygen double bond to ultimately furnish the alkanol, particularly 6-undecanol when the **K1** is undecanone. The corresponding linear alkanol produced according to any aspect of the present invention may then be dehydrated to form the corresponding alkene at the suitable temperature and pressure. Increasing the temperature and pressure would result in the dehydration of the ketone to the corresponding alkene. It would be within the knowledge of a skilled person to determine the suitable conditions for the use of the different ketonization catalysts in the method according to any aspect of the present invention. In particular, the ketonization catalyst may be a zirconia catalyst and may be used in the conversion of a ketone to the corresponding alkene. In particular, the temperature has to be at least 350°C for the ketone to be converted to the corresponding alkene. More in particular, the temperature may be maintained at about 350, 355, 360, 365, 370, 375, 380, 385, 390, 395, or 400°C during the method according to any aspect of the present invention. Even more in particular, the reaction temperature may be 350-420, 350-415, 350-410, 350-405, 350-400, 350-395, 350-390, 350-385, 350-380, 350-375, 350-370, 350-365, 350-360, 355-420, 355-415, 355-410, 355-405, 355-400, 355-395, 355-390, 355-385, 355-380, 355-375, 355-370, 355-365, 355-360, 360-420, 360-415, 360-410, 360-405, 360-400, 360-395, 360-390, 360-385, 360-380, 360-375, 360-370, 360-365, 365-420, 365-415, 365-410, 365-405, 365-400, 365-395, 365-390, 365-385, 365-380, 365-375, 365-370, 370-420, 370-415, 370-410, 370-405, 370-400, 370-395, 370-390, 370-385, 370-380, 370-375, 375-420, 375-415, 375-410, 375-405, 375-400, 375-395, 375-390, 375-385, 375-380, 380-420, 380-415, 380-410, 380-405, 380-400, 380-395, 380-390, 380-385, 385-420, 385-415, 385-410, 385-405, 385-400, 385-395, 385-390, 390-420, 390-415, 390-410, 390-405, 390-400, 390-395, 395-420, 395-415, 395-410, 395-405, 395-400, 400-420, 400-415, 400-410, or 400-405 °C. More in particular, the method according to any aspect of the present invention is carried out at a temperature between 350°C and 390°C. Even more in particular, alkenes are produced from ketones using the catalyst composition **X** at reaction temperatures of 350°C - 390°C.

In particular, at least for the alkene production from the ketone **K1**, the method is carried on in a reactor and the gases in the reactor are moving at gas hourly space velocity (GHSV) of 1 to 1000 m³/(m x h). More in particular, reactor may be a heated continuous flow-bed reactor. The GHSV refers to the speed of gases moving in the reactor per hour, particularly to the speed of gases moving over the catalyst. In particular, the gases in the reactor are at least ketone and/or the hydrogen releasing agent. More in particular, the gases in the reactor are at least ketone and hydrogen. The GHSV is 2 to 1000, 3 to 1000, 4 to 1000, 5 to 1000, 10 to 1000, 15 to 1000, 20 to 1000, 25 to 1000, 30 to 1000, 35 to 1000, 40 to 1000, 45 to 1000, 50 to 1000, 55 to 1000, 60 to 1000, 65 to 1000, 70 to 1000, 75 to 1000, 80 to 1000, 85 to 1000, 90 to 1000, 95 to 1000, 100 to 1000, 5 to 900, 10 to 900, 15 to 900, 20 to 900, 25 to 900, 30 to 900, 35 to 900, 40 to 900, 45 to 900, 50 to 900, 55 to 900, 60 to 900, 65 to 900, 70 to 900, 75 to 900, 80 to 900, 85 to 900, 90 to 900, 95 to 900, 100 to 900, 5 to 800, 10 to 800, 15 to 800, 20 to 800, 25 to 800, 30 to 800, 35 to 800, 40 to 800, 45 to 800, 50 to 800, 55 to 800, 60 to 800, 65 to 800, 70 to 800, 75 to 800, 80 to 800, 85 to 800, 90 to 800, 95 to 800, 100 to 800, 5 to 700, 10 to 700, 15 to 700, 20 to 700, 25 to 700, 30 to 700, 35 to 700, 40 to 700, 45 to 700, 50 to 700, 55 to 700, 60 to 700, 65 to 700, 70 to 700, 75 to 700, 80 to 700, 85 to 700, 90 to 700, 95 to 700, 100 to 700, 5 to 600, 10 to 600, 15 to 600, 20 to 600, 25 to 600, 30 to 600, 35 to 600, 40 to 600, 45 to 600, 50 to 600, 55 to 600, 60 to 600, 65 to 600, 70 to 600, 75 to 600, 80 to 600, 85 to 600, 90 to 600, 95 to 600, 100 to 600, 5 to 500, 10 to 500, 15 to 500, 20 to 500, 25 to 500, 30 to 500, 35 to 500, 40 to 500, 45 to 500, 50 to 500, 55 to 500, 60 to 500, 65 to 500, 70 to 500, 75 to 500, 80 to 500, 85 to 500, 90 to 500, 95 to 500, or 100 to 500 m³/(m x h). In particular, the GHSV is 5 to 500, 5 to 400, 10 to 400, 15 to 400, 20 to 400, 25 to 400, 30 to 400, 35 to 400, 40 to 400, 45 to 400, 50 to 400, 55 to 400, 60 to 400, 65 to 400, 70 to 400, 75 to 400, 80 to 400, 85 to 400, 90 to 400, 95 to 400, or 100 to 400, 5 to 300, 10 to 300, 15 to 300, 20 to 300, 25 to 300, 30 to 300, 35 to 300, 40 to 300, 45 to 300, 50 to 300, 55 to 300, 60 to 300, 65 to 300, 70 to 300, 75 to 300, 80 to 300, 85 to 300, 90 to 300, 95 to 300, or 100 to 300, 5 to 200, 10 to 200, 15 to 200, 20 to 200, 25 to 200, 30 to 200, 35 to 200, 40 to 200, 45 to 200, 50 to 200, 55 to 200, 60 to 200, 65 to 200, 70 to 200, 75 to 200, 80 to 200, 85 to 200, 90 to 200, 95 to 200, or 100 to 200, 5 to 150, 10 to 150, 15 to 150, 20 to 150, 25 to 150, 30 to 150, 35 to 150, 40 to 150, 45 to 150, 50 to 150, 55 to 150, 60 to 150, 65 to 150, 70 to 150, 75 to 150, 80 to 150, 85 to 150, 90 to 150, 95 to 150, or 100 to 150 m³/(m x h). In particular, the GHSV is 5 to 500 m³/( m³/(m x h). More in particular, the GHSV is 10 to 150 m³/(m³ x h). It is especially advantageous when the gases in the reactor are moving at this speed as then the ketone has sufficient time to be in contact with the catalyst and be converted into the alkene. The GHSV of the gaseous ketone may be measured using any means known in the art. In particular, the GHSV may be measured using a flow indicator. Namely, an indicator that can be used to measure the gas volume or the mass of the gases in the reactor where the method according to any aspect of the present invention is carried out.

A hydrogen releasing agent or hydrogen may be present at least during the alkene production from the ketone **K1**. The hydrogen releasing agent may be any compound that donates a H atom to the ketone to produce the corresponding alcohol and from there the alkene. In particular, the hydrogen releasing agent may be hydrogen or at least one carboxy acid or organic acid. More in particular, the hydrogen releasing agent is at least one alkanoic acid comprising 1 to 22 carbon atoms. The hydrogen releasing agent may therefore be at least one alkanoic acid comprising 1 to 22 carbon atoms or hydrogen. In particular the alkanoic acid is selected from straight chain alkanoic acids comprising 4 to 18, preferably 5 to 12, carbon atoms. More in particular, the alkanoic acid is hexanoic acid.

The hydrogen releasing agent may be supplied during the process of ketone **K1** production.

In one example, where the ketone **K1** undecanone, the corresponding alkanol is 6-undecanol and the corresponding alkene is 5-undecene.

The corresponding alkene is then converted to the respective alkane **A1**, in the presence of at least one hydrogenation metal catalyst for catalytic hydrogenation of the alkene to a corresponding alkane **A1**. Any hydrogenation metal catalyst may be used to carry out this step of alkane **A1** production.

In particular, the hydrogenation metal catalyst may be a homogeneous or heterogeneous catalyst. Homogeneous metal catalysts may be metal complexes that are known in the art. In particular, the hydrogenation metal catalyst may be a heterogeneous catalyst. Some advantages of using multiphase catalytic reactions using solid catalysts include easy separation of catalysts and products, easy recovery, and catalyst recycling, and relatively mild operating conditions. There are also clear economic and environmental incentives in using heterogeneous catalysts. In particular, the hydrogenation metal catalyst may be selected from the group consisting of ruthenium (Ru) catalyst, rhenium (Re) catalyst, nickel (Ni) catalyst, iron (Fe), cobalt (Co), palladium (Pd) catalyst and platinum (Pt) catalyst. More in particular, the catalyst may be selected from the group consisting of Ni, Pd and Pt catalyst. In one example, the hydrogenation metal catalyst used according to any aspect of the present invention may be nickel nanoparticles as described in Alonso, F. Tetrahedron, 2008, 64: 1847-52. In another example, Iron(II) PNP Pincer Complexes may be used as the hydrogenation metal catalyst for hydrogenation of the alkene to alkane, as disclosed in Gorgas, N., Organometallics, 2014, 33 (23): 6905-6914. In yet another example, magnetite nanoparticles of ruthenium (Ru) catalyst, rhenium (Re) catalyst, nickel (Ni) catalyst, iron (Fe), cobalt (Co), palladium (Pd) catalyst or platinum (Pt) catalyst as described in Tariq Shah M., et al., ACS Applied Materials & Interfaces, 2015: 7(12), 6480-9 may be used as the heterogenous hydrogenation metal catalyst according to any aspect of the present invention.

In yet another example, a copper-phosphine complex is used as a homogeneous hydrogenation metal catalyst according to any aspect of the present invention as disclosed in Chen, J-X., Tetrahedron, 2000, 56: 2153-2166. In a further example, a heterogenous Pt catalyst, in particular a Pt/Al2O3 catalyst, as disclosed in Journal of Molecular Catalysis A: 30 Chemical, 2014, 388-389: 116-122 may be used in hydrogenation of the alkene in to alkane. ChemSusChem, 2017: 10(11), 2527-2533 also discloses a variety of heterogenous catalysts such as Pt/C, Ru/C, and Pd/C that may be used in combination with or without an acid catalyst for the hydrogenation of alkene to alkane. Based on the above, a skilled person may determine a suitable hydrogenation catalyst to be used according to any aspect of the present invention to yield alkane **A1** from the alkene.

The hydrogenation metal catalyst used in the step of alkane **A1** production from alkene is preferably selected from the group consisting of ruthenium (Ru) catalyst, rhenium (Re) catalyst, nickel (Ni) catalyst, iron (Fe), cobalt (Co) and platinum (Pt) catalyst.

The final product from the method according to any aspect of the present invention in the presence of the catalyst composition **X** and a hydrogenation catalyst is therefore an alkane **A1** .

Some advantages of using multiphase catalytic reactions using the solid catalyst of the catalyst composition **X** include easy separation of catalysts and products, easy recovery, and catalyst recycling, and relatively mild operating conditions. There are also clear economic and environmental incentives in using the catalyst composition **X**.

A skilled person would easily be able to determine and vary the conditions, particularly temperature, pressure and reaction time accordingly to efficiently produce a ketone **K1** and/or alkane **A1** starting from carbonic acid according to any aspect of the present invention.

According to a further aspect of the present invention, there is provided a ketone **K1** of formula **I** produced from the method according to any aspect of the present invention.

R1-C(=O)-R2 Formula **I**,

wherein R1 and R2 is independently a hydrocarbon, preferably R is an organic radical selected from the group comprising unsubstituted and mono- or polysubstituted, branched and straight-chain alkyl radicals, cycloalkyl radicals, alkenyl radicals having one or more double bonds, alkynyl radicals having one or more triple bonds, aryl radicals, alkylaryl radicals, arylalkyl radicals, arylalkenyl radicals, alkyloxyalkyl radicals, hydroxyalkyl radicals and alkylthioalkyl radicals.

According to yet another aspect of the present invention, there is provided an alkane **A1** produced from the method according to any aspect of the present invention. Catalytic hydrogenation in the presence of the catalyst composition **X** results in the alkene being converted to form the corresponding linear alkane. The catalyst composition **X** is thus capable of providing dehydrating and/ or hydrogenating properties in any reaction.

According to a further aspect of the present invention, there is provided a use of a catalyst composition **X** to produce a ketone **K1** of the Formula **I**:

R1-C(=O)-R2 Formula **I,**

wherein a first carboxylic acid R1-COOH and a second carboxylic acid R2-COOH are reacted in the presence of a catalyst composition **X** to give **K1** and CO₂; and
wherein R1 and R2 is independently a hydrocarbon group, preferably R1 and R2 is an organic radical selected from the group consisting of unsubstituted and mono- or polysubstituted, branched and straight-chain alkyl radicals, cycloalkyl radicals, alkenyl radicals having one or more double bonds, alkynyl radicals having one or more triple bonds, aryl radicals, alkylaryl radicals, arylalkyl radicals, arylalkenyl radicals, alkyloxyalkyl radicals, hydroxyalkyl radicals, aminoalkylradicals and alkylthioalkyl radicals; and wherein the catalyst composition **X** comprises:
   - at least one precious metal; and/or
   - at least one mixed oxide.

According to a further aspect of the present invention, there is provided a use the catalyst composition **X** to produce an alkane **A1**.

In one example, the ketone **K1** is undecanone, the carboxylic acid is hexanoic acid and the alkane **A1** is undecane.

Unless stated otherwise, all percentages (%) given are percentages by mass.

The examples adduced hereinafter describe the present invention by way of example, without any intention that the invention, the scope of application of which is apparent from the entirety of the description and the claims, be restricted to the embodiments specified in the examples.

### EXAMPLES

### Example 1

The reaction was conducted in a heated continuous flow-bed reactor. Gaseous hexanoic acid (1.2 m³/h) was continuously fed to a reactor charged with zirconium oxide on silica (95 wt.%, 10.15 kg) at 343°C. The gaseous out stream was collected and analyzed by gas chromatography (GC) for its composition. In total, 3 kg of hexanoic acid was fed to the reactor, leading to a 2.2 kg mixture consisting of 99% 6-undecanone and <1% hexanoic acid, alongside 0,6 kg of carbon dioxide and 0,2 kg water.

### Example 2

The reaction was conducted in a heated continuous flow-bed reactor. Gaseous hexanoic acid (1.2 m³/h) was continuously fed to a reactor charged with zirconium oxide on silica (95 wt.%, 10.2 kg) at 365°C. The gaseous out stream was collected and analyzed by nuclear magnetic resonance spectroscopy (NMR) for its composition. In total, 3 kg of hexanoic acid was fed to the reactor, leading to a 2.3 kg mixture consisting of 15% undecene and 75% 6-undecanone and 10% of byproducts, alongside 0,5 kg of carbon dioxide and 0,2 kg water. The residual amount of hexanoic acid was lower than the detection limit.

## Claims

1. A method of producing a ketone **K1** of the Formula **I:**
R1-C(=O)-R2 Formula **I,**
wherein a carboxylic acid R1-COOH and a carboxylic acid R2-COOH are reacted in the presence of a catalyst composition **X** to give **K1** and CO₂; and
wherein R1 and R2 is independently a hydrocarbon group, preferably R1 and R2 is an organic radical selected from the group consisting of unsubstituted and mono- or polysubstituted, branched and straight-chain alkyl radicals, cycloalkyl radicals, alkenyl radicals having one or more double bonds, alkynyl radicals having one or more triple bonds, aryl radicals, alkylaryl radicals, arylalkyl radicals, arylalkenyl radicals, alkyloxyalkyl radicals, hydroxyalkyl radicals, aminoalkylradicals and alkylthioalkyl radicals; and
wherein the catalyst composition **X** comprises:
- at least one precious metal; and/or
- at least one mixed oxide.

2. The method according to claim 1, wherein the mixed oxide comprises zirconium dioxide and silicon dioxide;
wherein the precious metal is supported and the support is not entirely made of the mixed oxide; and
wherein a mass ratio of zirconium dioxide to silicon dioxide in the mixed oxide is 86:14 to 99.9:0.1.

3. The method according to either claim 1 or 2, wherein the catalyst composition **X** comprises at least one mixed oxide of zirconium dioxide and silicon dioxide; and
wherein a mass ratio of zirconium dioxide to silicon dioxide in the mixed oxide is 86:14 to 99.9:0.1.

4. The method according to any one of the preceding claims, wherein the carboxylic acids are reacted in the presence of the catalyst composition **X** at a temperature of at least 300°C.

5. The method according to any one of the preceding claims, wherein the carboxylic acid is an alkanoic acid comprising 1 to 22 carbon atoms, preferably a hexanoic acid.

6. The method according to any one of the preceding claims, wherein the ketone **K1** is undecanone and the carboxylic acid is hexanoic acid.

7. The method according to any one of the preceding claims, wherein the support in the catalyst composition **X** does not comprise the mixed oxide.

8. The method according to any one of the preceding claims, wherein under suitable conditions, the ketone **K1** is converted to the corresponding alkane **A1** in the presence of the catalyst composition **X** and a hydrogenation catalyst.

9. A ketone **K1** of formula I produced from the method according to any one of the claims 1 to 7:
R1-C(=O)-R2 Formula **I,**
wherein R1 and R2 is independently a hydrocarbon, preferably R is an organic radical selected from the group comprising unsubstituted and mono- or polysubstituted, branched and straight-chain alkyl radicals, cycloalkyl radicals, alkenyl radicals having one or more double bonds, alkynyl radicals having one or more triple bonds, aryl radicals, alkylaryl radicals, arylalkyl radicals, arylalkenyl radicals, alkyloxyalkyl radicals, hydroxyalkyl radicals and alkylthioalkyl radicals.

10. An alkane **A1** produced from the method according to claim 8.

11. Use of a catalyst composition **X** to produce a ketone **K1** of the Formula **I**:
R1-C(=O)-R2 Formula **I,**
wherein a first carboxylic acid R1-COOH and a second carboxylic acid R2-COOH are reacted in the presence of a catalyst composition **X** to give **K1** and CO₂; and
wherein R1 and R2 is independently a hydrocarbon group, preferably R1 and R2 is an organic radical selected from the group consisting of unsubstituted and mono- or polysubstituted, branched and straight-chain alkyl radicals, cycloalkyl radicals, alkenyl radicals having one or more double bonds, alkynyl radicals having one or more triple bonds, aryl radicals, alkylaryl radicals, arylalkyl radicals, arylalkenyl radicals, alkyloxyalkyl radicals, hydroxyalkyl radicals, aminoalkylradicals and alkylthioalkyl radicals; and
wherein the catalyst composition **X** comprises:
- at least one precious metal; and/or
- at least one mixed oxide.

12. Use of a catalyst composition **X** and a hydrogenation catalyst to produce an alkane **A1** wherein the catalyst composition **X** comprises:
- at least one precious metal; and/or
- at least one mixed oxide.

13. Use according to either claim 11 or 12, wherein the mixed oxide comprises zirconium dioxide
and silicon dioxide; and
wherein the precious metal is supported and the support is not entirely made of the mixed oxide, preferably the support of the precious metal is selected from the group consisting of silicon dioxide, aluminum oxide and mixtures thereof, preferably the precious metal is selected from the group consisting of ruthenium, rhodium, palladium, osmium, iridium, platinum and mixtures thereof, more preferably palladium; and
wherein a mass ratio of zirconium dioxide to silicon dioxide in the mixed oxide is 86:14 to 99.9:0.1.

14. Use according to any one of the claims 11 or 13, wherein the catalyst composition **X** comprises at least one mixed oxide of zirconium dioxide and silicon dioxide; and
wherein a mass ratio of zirconium dioxide to silicon dioxide in the mixed oxide is 86:14 to 99.9:0.1.

15. Use according to to any one of the preceding claims 11 to 14, wherein the ketone K1 is undecanone, the carboxylic acid is hexanoic acid and the alkane A1 is undecane.
